# EUROPÄISCHE PATENTSCHRIFT

(11) **EP 1 407 263 B1**
(45) Veröffentlichungstag und Bekanntmachung des Hinweises auf die Patenterteilung: **16.04.2008**
(21) Anmeldenummer: 02735058.6
(22) Anmeldetag: 03.05.2002
(51) Int. Cl.: G01N 33/50

(54) **VERFAHREN ZUR EXTRAKORPORALEN QUALITATIVEN UND/ODER QUANTITATIVEN ERFASSUNG NEUROTOXISCHER SUBSTANZEN IM BLUTPLASMA EINES INDIVIDUUMS**
METHOD FOR THE EXTRA-CORPOREAL QUALITATIVE AND/OR QUANTITATIVE RECORDING OF NEURO-TOXIC SUBSTANCES IN THE BLOOD PLASMA OF AN INDIVIDUAL
PROCEDE DE DETECTION EXTRACORPORELLE QUALITATIVE ET/OU QUANTITATIVE DE SUBSTANCES NEUROTOXIQUES DANS LE PLASMA SANGUIN D'UN INDIVIDU

(30) Priorität: 22.06.2001 DE 10129693
(43) Veröffentlichungstag der Anmeldung: 14.04.2004
(73) Patentinhaber: Loock, Jan, 24576 Bad Bramstedt (DE); Mitzner, Steffen, Dr. med., D-18119 Rostock (DE); Weiss, Dieter G., D-18055 Rostock (DE); Stange, Jan, D-18059 Rostock (DE)
(72) Erfinder: LOOCK, Jan, 18147 Rostock (DE); MITZNER, Steffen, R., 18119 Rostock (DE); WEISS, Dieter, G., 18055 Rostock (DE); STANGE, Jan, 18059 Rostock (DE); GROSS, Günter, W., Denton, TX 76205 (US); GRAMOWSKI, Alexandra, 18055 Rostock (DE); SCHIFFMANN, Dietmar, 18057 Rostock (DE)
(74) Vertreter: Weber, Roland
(86) Internationale Anmeldenummer: PCT/DE2002/001609
(87) Internationale Veröffentlichungsnummer: WO 2003/001201

(56) Entgegenhaltungen:
- DE-A- 19 932 720
- US-A- 5 480 554
- LOOCK JAN ET AL: "Reaction of spontaneously active neuronal networks to ultrafiltrates of human blood plasma in vitro - A possible biosensor of cerebral function during metabolic diseases?" EUROPEAN JOURNAL OF CELL BIOLOGY, Bd. 78, Nr. SUPPL. 49, 1999, Seite 82 XP009009795 23rd Annual Meeting of the German Society for Cell Biology;Rostock, Germany; March 14-18, 1999 ISSN: 0171-9335
- LOOCK JAN ET AL: "The effect of albumin dialysis (MARS) on neuronal network activity evaluated in vitro." HEPATOLOGY, Bd. 30, Nr. 4 PART 2, Oktober 1999 (1999-10), Seite 163A XP009009796 50th Annual Meeting and Postgraduate Courses of the American Association for the Study of Liver Diseases;Dallas, Texas, USA; November 5-9, 1999 ISSN: 0270-9139
- YURDAYDIN C ET AL: "OPIOID RECEPTOR LIGANDS IN HUMAN HEPATIC ENCEPHALOPATHY" JOURNAL OF HEPATOLOGY, MUNKSGAARD INTERNATIONAL PUBLISHERS, COPENHAGEN, DK, Bd. 29, Nr. 5, 1998, Seiten 796-801, XP000857395 ISSN: 0168-8278
- JALAN R ET AL: "Hepatic encephalopathy and ascites" LANCET, XX, XX, Bd. 350, Nr. 9087, 1. November 1997 (1997-11-01), Seiten 1309-1315, XP004264968 ISSN: 0140-6736
- GROSS GUENTER W ET AL: "Odor, drug and toxin analysis with neuronal networks in vitro: Extracellular array recording of network responses." BIOSENSORS & BIOELECTRONICS, Bd. 12, Nr. 5, 1997, Seiten 373-393, XP002239346 ISSN: 0956-5663
- GROSS GUENTER W ET AL: "The use of neuronal networks on multielectrode arrays as biosensors." BIOSENSORS & BIOELECTRONICS, Bd. 10, Nr. 6-7, 1995, Seiten 553-567, XP002239347 ISSN: 0956-5663

## Beschreibung

Die Erfindung betrifft ein Verfahren zur extrakorporalen qualitativen und/ oder quantitativen Erfassung neurotoxischer Substanzen im Blutplasma eines Individuums, bei dem man ein System mit auf einem Mikroelektroden-Array bzw. einem Neurochip kultivierten oder aufgebrachten Neuronen oder neuronalen Netzwerken und einer Vorrichtung zum Messen der elektrischen Aktivität wenigstens eines oder mehrerer der Neuronen bereitstellt, die Neuronen mit einer aufbereiteten Plasmaprobe in Berührung bringt und die elektrische Aktivität der Neuronen mißt.

### Stand der Technik

Eine Vielzahl von Erkrankungszuständen in der Medizin kann mit einer Einschränkung des Bewusstseins der Patienten bis hin zum Koma einhergehen. Oft geht diese Einschränkung auf eine Veränderung der Zusammensetzung des Blutplasmas zurück (metabolische Encephalopathien, z.B. im Rahmen eines Leber-oder Nierenversagens, diabetisches Koma, Vergiftungszustände, Medikamentenwirkung).

Unter den metabolischen Encephalopathien, die durch den weitgehenden Ausfall wichtiger Entgiftungssysteme des Körpers wie Leber oder Niere verursacht werden, besitzt die hepatische Encephalopathie aufgrund der Häufigkeit des Auftretens schwerer Lebererkrankungen und bisher noch unzureichender therapeutischer Möglichkeiten große medizinische und sozioökonomische Relevanz. Die genauen Mechanismen ihrer Entstehung sind hingegen noch weitgehend ungeklärt. Nach gegenwärtigem Kenntnisstand wird ursächlich eine Beeinflussung der zentralnervösen Funktion durch im Leberversagen akkumulierende Metabolite angenommen, verursacht durch Funktionsverluste von für die Ausschleusung oder Entgiftung von Stoffwechselendprodukten wichtigen Organen, wie Leber oder Niere. Im Verlauf kommt es zu einer progredienten Encephalopathie, die klinisch durch undulierende Alterationen von Wachheitsgrad, Aufmerksamkeit und Konzentrationsvermögen sowie extrapyramidale motorische Störungen, wie gesteigertem Muskeltonus und Tremor imponiert. Hinzu kommen Verwirrtheit, Fehlwahrnehmungen und Halluzinationen. In schweren Fällen führt dies bis zum Delirium, Koma und schließlich zum Tod des Patienten. Bezüglich der Mechanismen, die zur Funktionseinschränkung des zentralen Nervensystem führen, existieren verschiedene Hypothesen. Hierzu zählen die Theorie der falschen Neurotransmitter (Fischer, J.E., Baldessarini, R.J.: False neurotransmitters and hepatic failure. Lancet 2, (1975), 75-80), die Ammoniak-Hypothese (Blei, A.T., Olafsson, S., Therrien, G., Butterworth, R.F.: Ammonia-induced brain edema and intracranial hypertension in rats after portocaval anastomosis. Hepatology 19, (1994), 1437-1444; Butterworth, R.F.: Pathophysiology of hepatic encephalopathy: the ammonia hypothesis revisited. In: Progress in hepatic encephalopathy and metabolic nitrogen exchange. Hrsg.: Bengtsson, F., Boca Raton, CRC Press, 1991, 9-24), die GABA-Benzodiazepinrezeptor-Liganden-Hypothese (Basile, A.S., Harrison, P.M., Hughes, R.D., Gu, Z.Q., Pannell, L., McKinney, A., Jones, E.A., Williams, R.: Relationship between plasma benzodiazepine receptor ligand concentrations and severity of hepatic encephalopathy. Hepatology 19, (1994), 112-121) und die Synergismus-Theorie über das sich gegenseitig verstärkende Wirken mehrerer verantwortlicher Metaboliten (Zieve L.: Role of toxins and synergism in hepatic encephalopathy. In: Hepatic encephalopathy. Hrsg. Butterworth, R.F., Humana Press, Clifton, New Jersey, 1989, 141-156). Bei den bisher identifizierten potentiellen Toxinen handelt es sich um Ammoniak, Bilirubin, Gallensäuren, Tryptophan, phenolische und indolische Abbauprodukte von Aminosäuren, Mercaptane, mittelkettige Fettsäuren und endogene Benzodiazepine (Weissenborn, K.: Recent developments in the pathophysiology and treatment of hepatic encephalopathy. Baillière's Clin. Gastroenterol. 6, (1992), 609-630). Den genannten Theorien gemeinsam ist, daß die infolge des Organversagens im Körper akkumulierenden Substanzen eine zentrale Rolle in der Pathogenese der hervorgerufenen Encephalopathie besitzen (Jones, E.A., Gammal, S.H.: Hepatic encephalopathy. In: The liver: Biology and Pathobiology, 2. Auflage, Hrsg.: Arias, I.M., Raven Press, Ltd., New York, 1988, 985-1004; Record, C.O.: Neurochemistry of hepatic encephalopathy. Gut 32, (1991), 1261-1263). Ursache für die noch bestehende Unklarheit über die im Hirn ablaufenden pathophysiologischen Prozesse ist vor allem die schwierige schlüssige Beweisführung an in vivo-Modellen. Auch die genauen Entstehungsmechanismen vieler anderer Komazustände sind noch weitgehend ungeklärt. Oft korreliert die Messung einzelner Laborparameter, die nach gegenwärtigem Wissensstand als pathogenetisch bedeutsam angesehen werden, nicht mit dem klinisch bestehenden Grad der Bewusstseinseinschränkung. Der Vergleich der Effekte von Plasmaproben von Patienten mit den Wirkungen verschiedener Reinsubstanzen kann zur Identifizierung möglicher Auslöser des Komas und damit zur Aufklärung der Pathogenese dieser Erkrankungsformen beitragen.

Insbesondere bei Komazuständen unklarer Art, bei denen es sich um Vergiftungen oder Suizidversuche handeln könnte, besteht im Rahmen der Notfallmedizin bei vitaler Bedrohung des Patienten häufig die Notwendigkeit einer möglichst unmittelbaren Erkennung und Quantifizierung des auslösenden Giftstoffes zur Einleitung einer optimal ausgerichteten Behandlungsweise.

Unter der Bezeichnung MARS (Molecular Adsorbents Re-circulating-System, Teraklin AG, Rostock) wird ein neuartiges Dialyseverfahren zur extrakorporalen Detoxikation eingesetzt, das speziell zur Elimination toxischer Verbindungen aus dem Plasma von Patienten im Leberversagen entwickelt wurde (Stange, J., Mitzner, S., Ramlow, W., Gliesche H., Hickstein, H., Schmidt, R.: A new procedure for the removal of protein-bound drugs and toxins. ASAIO J. 39, (1993), M621-M625). Hierbei handelt es sich neben wasserlöslichen Metaboliten auch um relativ lipophile Substanzen. Letztere werden im Blut primär an Plasmaeiweiße - vor allem an Albumin - gebunden transportiert. MARS ermöglicht durch das Vorhandensein von Akzeptorproteinen (Albumin) im Dialysat die Elimination dieser ansonsten kaum dialysablen Verbindungen aus dem Blutplasma (Stange, J., Ramlow, W., Mitzner, S., Schmidt, R., Klinkmann, H.: Dialysis against a recycled albumin solution enables the removal of albumin-bound toxins. Artif. Org. 17, (1993), 809-813; Stange, J., Mitzner, S.R., Risler, T., Erley, C.M., Lauchart, W., Göhl, H., Klammt, S., Peszynski, P., Freytag, J., Hickstein, H., Löhr, M., Liebe, S., Schareck, W., Hopt, U.T., Schmidt, R.: Molecular Adsorbents Recirculating System (MARS): Clinical results of a new membrane-based blood purification system for bioartificial liver support. Artif. Org. 23, (1999), 319-330). Zudem wird hierdurch auch eine günstige Beeinflussung der ansonsten bei diesem Krankheitsbild typischen Aminosäuredysbalance zwischen verzweigtkettigen und aromatischen Aminosäuren im Plasma erreicht (Loock J, Peters E, Stange J, Mitzner S, Peszinski P, Klammt S, Liebich H, Schmidt R: Change of human serum albumin amino acid patterns (Fischer-index) during a new dialysis treatment for liver failure (MARS). Internat J Artif Org 20(1997) 500). Im Rahmen des klinischen Einsatzes des Verfahrens an Patienten im Leberkoma in Studien zeigte sich eine signifikante Minderung des Encephalopathiegrades der Patienten (Stange J, Mitzner S, Klammt S, Freytag J, Peszynski P, Loock J, Hickstein H, Korten G, Schmidt R, Hentschel J, Schulz M, Löhr M, Liebe S, Schareck W, Hopt UT: Liver Support by Extracorporeal Blood Purification-A Clinical Observation. Liver Transpl. 2000 Sep;6(5):603-13).

Die klinische Einschätzung des Grades der auf das Leberversagen zurückgehenden hepatischen Encephalopathie wird jedoch unter Umständen durch das Vorhandensein anderer komaauslösender Faktoren oder Wirkungen von Medikamenten erschwert oder gänzlich unmöglich. Ein Verfahren zur schnellen und zuverlässigen Kontrolle des Behandlungserfolges, d. h. des Grades der Detoxikation während und nach der Behandlung fehlt bislang.

Angesichts der oft vorhandenen Multimorbidität dieser Hochrisikopatienten können Störungen der Funktion des Zentralnervensystems auch auf zusätzlich vorhandene andere Erkrankungen zurückgehen. In diesem Rahmen ist auch die Stellung der richtigen Diagnose oft nur verzögert möglich. Konkrete Beispiele für andersartige Auslöser zerebraler Funktionsstörungen können in diesem Zusammenhang Infektionen (z.B. Meningitiden, Abszesse, Encephalitis) oder Nebenwirkungen von Medikamenten aber auch Durchblutungsstörungen des Gehirns oder Akkumulation nierenpflichtiger Metaboliten im Rahmen eines komplizierenden Nierenversagens sein. Eine verbesserte Diagnostik , die eine rechtzeitige Erkennung einer anderen zur Bewußtseinstrübung führenden Ursache ermöglicht, würde durch frühzeitige Einleitung der adäquaten Therapie die Prognose der Patienten verbessern und die Verweildauer in hoch kostenintensiven Intensivstationen verkürzen.

Die Entwicklung neuronaler Netzwerke auf Mikroelektroden-Arrays ermöglicht die kontinuierliche Registrierung und Analyse der elektrischen Aktivität von Neuronenverbänden unter unterschiedlichen experimentellen Bedingungen (Gross, G.W., Rieske, E., Kreutzberg, G.W., Meyer, A.: A new fixed-array multimicroelectrode system designed for longterm monitoring of extracellular single unit neuronal network activity in vitro. Neurosci. Lett. 6, (1977), 101-105; Gross, G.W.: Internal dynamics of randomized mammalian neuronal networks in culture. In: Enabling technologies for cultured neural networks. Hrsg.: Stenger, McKenna, T.M., Academic Press, New York, 1994, 277-317). Bisherige Arbeiten zeigen, daß die Netzwerkaktivität durch die Applikation einer Vielzahl von neuronal aktiven Substanzen jeweils in konzentrations- und substanzspezifischer und im allgemeinen reversibler Weise beeinflußt wird. Erst zelltoxische Konzentrationen terminieren die Netzwerkaktivität irreversibel (Gross, G.W.: Internal dynamics of randomized mammalian neuronal networks in culture. In: Enabling technologies for cultured neural networks. Hrsg.: Stenger, McKenna, T.M., Academic Press, New York, 1994, 277-317). Hierdurch ergeben sich völlig neuartige Möglichkeiten in der qualitativen und quantitativen Erfassung der pathophysiologischen Abläufe im Gehirn.

Loock et al., European Journal of Cell Biology (1999) Bd. 78, Nr. 49, S. 82, und Loock et al., Hepatology (1999) Bd. 30, Nr. 4, Part 2, S. 163A, offenbaren die Verwendung eines Ultrafiltrats von menschlichem Blutplasma für den Kontakt mit neuronalen Netzwerken in vitro zur Detektion von Toxinen im Blut von Patienten mit Leberkoma.

Jalan und Hayes, Lancet (1997) Bd. 350, Nr. 9087, S. 1309-1315, beschreiben, daß die meisten Moleküle, die mit dem Entstehen von hepatischer Encephalopathie in Verbindung gebracht werden, niedermolekulare Stoffe sind, wie z.B. Ammoniak, Merkaptane, Phenole, Neurotransmitter etc.

Yurdaydin et al., Journal of Hepatology (1998) Bd. 29, Nr. 5, S. 796-801, beschreiben die Messung der Konzentrationen der Opioidliganden Met-Enkephalin, Leu-Enkephalin und Beta-Endorphin im Plasma und in cerebrospinaler Flüssigkeit von Patienten mit hepatischer Encephalopathie. Die Messungen werden mit dafür kommerziell erhältlichen Antikörpertests durchgeführt.

Die DE 19932720 A1 beschreibt ein Verfahren zur Messung der Wirkung von Stoffen, wie pharmakologischen Substanzen und Toxinen, auf einen neuronalen Zellverband. Bei dem Verfahren werden Neuronen in Lösung auf einen Multielektroden-Array (Neurochip) aufgebracht, die bioelektrische Aktivität des Zellverbandes gemessen und über Veränderung mindestens einer Ionenkonzentration in der extrazellulären Lösung ein definierter Aktivitätszustand eingestellt. Anschließend wird der zu untersuchende Stoff zu der extrazellulären Lösung zugegeben und die Aktivität des Zellverbandes bzw. deren Änderung erneut gemessen. Das Verfahren soll ein Screening von Substanzen hinsichtlich ihrer Wirksamkeit auf neuronale Erregbarkeit, synaptische Übertragung und/oder Netzwerkverhalten erlauben. Da die Aktivitäts-Zustände auf der Interaktion von zellulären und synaptischen Mechanismen beruhen, reagieren die Zellverbände sehr sensibel auf die Wirkung von extrazellulär zugegebenen Substanzen. Hierdurch lässt sich die Wirkung des zu testenden Stoffes bzw. der zu testenden Substanz sehr gut und insbesondere reproduzierbar testen. Zusätzlich erlaubt die Kenntnis der Abhängigkeiten der Netzwerkzustände von extrazellulär manipulierbaren Größen (neuronale Erregbarkeit und Wirksamkeit der synaptischen Übertragung) eine gute Kalibrierung und damit das Einstellen eines definierten Testzustandes. Die DE 19932720 A1 bezieht sich im wesentlichen auf die Herstellung und die Kalibrierung des Systems. Als einzige Testsubstanz wurde eine 100 µM Lösung von 4-Aminopyridin eingesetzt.

Es hat sich jedoch gezeigt, daß sich das Verfahren gemäß der DE 19932720 A1 zur Erfassung neurotoxischer Substanzen im Blutplasma oder Serum von Patienten nicht ohne weiteres in reproduzierbarer Weise anwenden läßt. Im Gegensatz zu Reinsubstanzen, wie sie als Testsubstanz in der DE 19932720 A1 eingesetzt wurden, enthält Blutplasma ein Gemisch aus einer Vielzahl von Proteinen und anderen lipophilen und hydrophilen Substanzen. Die Proteine können als Träger für unterschiedliche Substanzen mit nur geringer Wasserlöslichkeit wirken und somit deren Transport im Blutkreislauf ermöglichen. Andere Eiweiße haben jedoch Aufgaben in der unspezifischen und spezifischen (immunologischen) Abwehr von im Organismus als fremd erkannten Stoffen und von Mikroorganismen (Komplementfaktoren, Immunglobuline). Da die neuronalen Netzwerke aus Föten von Säugetieren gewonnen werden (z.B. Maus), kommt es bei Zugabe von Blutplasma oder Serum zum Sensorsystem zum Kontakt der Plasmaproteine mit den Zellen einer fremden Species. Hierdurch kann es zu einer Aktivierung des Komplementsystems sowie spezifischen immunologischen Interaktionen kommen, in deren Folge die als fremd erkannten Zellen durch das Abwehrsystem attackiert und geschädigt werden.

Zudem sind die anderen lipophilen und hydrophilen Substanzen zum Teil im Plasma von encephalopathischen oder komatösen Patienten, z. B. im Leberversagen, in anderen Konzentrationen, d. h. höheren oder niedrigeren, oder in anderen Konfigurationen, Bindungs- oder Ladungszuständen vorhanden als im Plasma von Gesunden. Sie stellen damit potentielle Indikatoren für einen bestimmten Krankheitszustand dar. Andererseits ist jedoch auch eine Vielzahl von Substanzen im Plasma vorhanden, die für das spezielle Krankheitsbild keine spezifischen Indikatoren sind und/ oder die elektrische Aktivität der Neuronen in dem bekannten Testverfahren und damit auch die bei der Messung der Ströme erhaltenen Ergebnisse erheblich beeinflussen bzw. stören und sichere, reproduzierbare Aussagen über die Meßergebnisse sehr erschweren.

Die Aufgabe der vorliegenden Erfindung bestand daher darin, das an sich bekannte Verfahren zur Erfassung neurotoxischer Substanzen mittels eines neuronalen Netzwerkes in Kultur auf einem Mikroelektroden-Array so weiterzubilden und zu verbessern, daß es für die Erfassung neurotoxischer Substanzen im Blutplasma eines Individuums, insbesondere beim Vorliegen von encephalopathischen Zuständen, wie im Leberversagen, oder von Komazuständen anderer Ursache, zuverlässige und reproduzierbare Ergebnisse liefert.

Diese erfindungsgemäße Aufgabe wird durch ein Verfahren der eingangs genannten Art gelöst, das dadurch gekennzeichnet ist, daß man das Blutplasma des Individuums zur Aufbereitung vor der Messung einer Extraktion fettlöslicher Stoffe unter Verwendung eines organischen Lösungsmittels für fettlösliche Stoffe unterzieht und das Blutplasma des Individuums zur Aufbereitung vor der Messung gegebenenfalls einer Ultrafiltration unterzieht, wobei eine Ausschlußgrenze für das Molekulargewicht gewählt wird, die eine weitestgehende Abtrennung der Plasmaeiweiße von den anderen gelösten Substanzen bewirkt, und man den fettlösliche Stoffe enthaltenden Extrakt und gegebenenfalls das Ultrafiltrat im Gemisch mit dem Extrakt oder beides nacheinander für die Messung einsetzt.

Es konnte gezeigt werden (s. unten), daß in bestimmter Weise aufbereitete Blutplasmaproben von gesunden Individuen und komatösen Patienten, z. B. im Leberversagen, in reproduzierbarer Weise unterschiedliche elektrische Erregungszustände in neuronalen Netzwerken auf einem Mikroelektroden-Array (Neurochip) hervorriefen, und zwar in der Art, dass die von gesunden Individuen stammenden Proben jeweils keine oder nur gering ausgeprägte Veränderungen gegenüber der Ausgangsaktivität des neuronalen Netzwerkes bewirkten, während die Proben von komatösen Patienten im Leberversagen jeweils eine starke Änderung dieser Aktivität hervorriefen. Bei Verwendung von nicht nach dem erfindungsgemäßen Verfahren vorbehandeltem Blutplasma kam es bereits bei der Zugabe von Proben gesunder Individuen zu einer starken Beeinflussung der Aktivität des Zellverbandes, so daß die Unterschiede zu komatösen Patienten erheblich geringer ausgeprägt oder gar nicht erkennbar waren. Dies wurde darauf zurückgeführt, daß bestimmte Substanzen, die sowohl im Blut von gesunden Patienten als auch von kranken Patienten vorhanden sind, die elektrische Aktivität der Neuronen in gleicher Weise stark beeinflussen, so daß Reaktionen der Neuronen auf krankheitsspezifische Substanzen dadurch überlagert wurden. Interaktionen zwischen den oben genannten, im Plasma und Serum enthaltenen Proteinen zur Abwehr von Fremdstoffen und Krankheitserregern mit den Oberflächen des Zellverbandes haben sehr wahrscheinlich eine große Bedeutung bei der Auslösung dieser Effekte. Eine signifikante Unterscheidung zwischen Plasma von gesunden Individuen und komatösen Patienten war daher nicht oder nicht mit ausreichender Deutlichkeit für einen reproduzierbaren Einsatz des Verfahrens möglich.

Spezifische Änderungen der neuronalen Aktivität nach Zugabe einer aufbereiteten Plasma- oder Serumprobe des Patienten können wichtige Hinweise auf die zugrundeliegende Ursache des Komas geben und eine zielgerichtete weitere Diagnostik und Therapie ermöglichen. Durch die quantitative Erfassung der Änderung der neuronalen Aktivität nach Probenzugabe wird nicht nur eine Aussage über den Grad der vorhandenen Intoxikation sondern bei Untersuchung mehrerer Proben eines Patienten im Verlauf der Erkrankung auch eine Beurteilung der Wirksamkeit der angewendeten Therapieform ermöglicht.

Überraschenderweise hat sich gezeigt, daß mit Blutplasma, welches vor der Messung einer Ultrafiltration mit einer Ausschlußgrenze für das Molekulargewicht von 60.000 Dalton unterzogen worden war, eine deutlicher zu unterscheidende Neuronenerregung zwischen Plasma von gesunden Individuen und kranken Patienten lieferte. Noch deutlicher ausgeprägt waren die Ergebnisse bei einer Ausschlußgrenze für das Molekulargewicht von 45.000 Dalton und noch besser 30.000 Dalton.

Die z. B. mit Blutsplasma von Patienten im Leberversagen gemessenen Erregungsströme waren signifikant höher als diejenigen, die mit in gleicher Weise ultrafiltriertem Blutplasma von gesunden Individuen gemessen wurden. Es konnte auch gezeigt werden, daß die Stärke der Neuronenerregungen mit der Schwere des Krankheitsbildes korrelierten. Bei Plasmaproben von Patienten, deren Blut einer Leberdialyse, z. B. nach dem MARS-Verfahren, unterzogen wurde, beobachtete man eine kontinuierliche Abnahme der Erregungshöhe im Verlauf der Dialyse. Es konnte somit eine reproduzierbare Korrelation zwischen der Konzentration krankheitsspezifischer Substanzen im Blutplasma und der Höhe der Erregungsströme festgestellt werden. Des weiteren wurde festgestellt, daß der Verlauf der Erregungsströme während einer Messung bei Plasmaproben von verschiedenen Patienten mit dem gleichen Krankheitsbild über die Zeit der Messung sehr ähnlich war, d.h. ein vergleichbares Erregungsmuster aufwies. Anhand des Erregungsmusters, das von einer gemäß dem erfindungsgemäßen Verfahren ultrafiltrierten Blutplasmaprobe eines Patienten gemessen wurde, konnte somit durch Vergleich mit Standards aus gesunden Patienten sowie Standards aus Patienten mit bekanntem Krankheitsbild und bekannter Krankheitsschwere eine qualitative sowie quantitative Relation hergestellt werden.

Bei den hochmolekularen Substanzen, die durch Ultrafiltration von Blutplasma abgetrennt werden können, handelt es sich in der Hauptsache um höhermolekulare Proteine, wie z. B. Antikörper, Albumin usw. Bei der Abtrennung dieser Eiweiße durch Ultrafiltration wurden zum größten Teil relativ gut wasserlösliche Verbindungen im Ultrafiltrat erhalten, während fettlösliche Substanzen nur in geringem Maße enthalten waren. Dies wird darauf zurückgeführt, daß sich einige der fettlöslichen Substanzen an die hochmolekularen Proteine binden und dadurch bei der Ultrafiltration ebenfalls vom Plasma getrennt werden. Es konnte jedoch auch gezeigt werden, daß auch die fettlöslichen Substanzen im Blutplasma eigene Effekte auf die Aktivität der neuronalen Netzwerke in dem Verfahren der vorliegenden Erfindung bewirkten, die wiederum nur durch Proben von komatösen Patienten hervorgerufen wurden.

Erfindungsgemäß kann daher auch alleine oder in Kombination mit den erfindungsgemäß verwendeten ultrafiltrierten Plasmaproben ein Extrakt der fettlöslichen Substanzen aus dem Blutplasma eingesetzt werden. Hierzu wird das Blutplasma mit einem vorzugsweise organischen Lösungsmittel versetzt und extrahiert. In einer bevorzugten Ausführungsform der Erfindung wird hierfür Dichlormethan oder Chloroform eingesetzt. Nach guter Durchmischung des Blutplasmas mit dem organischen Lösungsmittel läßt man das Gemisch absetzen und die Phasen trennen.

Die organische Phase mit den darin gelösten fettlöslichen Substanzen wird abgenommen, das Lösungsmittel läßt man verdunsten und löst bzw. suspendiert den Rückstand mit den fettlöslichen Substanzen erneut, vorzugsweise in dem gleichen oder einem ähnlichen Medium, in dem sich die kultivierten neuronalen Netzwerke auf dem Mikroelektroden-Array befinden. Bevorzugt verwendet man zum Resuspendieren eine Mischung aus Zellkulturmedium und einer geringen Konzentration eines Lösungsvermittlers, wie Dimethylsulfoxid.

Bei einer besonders bevorzugten Ausführungsform der Erfindung wird das Blutplasma vor der Extraktion mit dem organischen Lösungsmittel angesäuert. Hierfür wird vorzugsweise eine anorganische Säure, wie Salzsäure, verwendet. Durch das Ansäuern werden saure organische Substanzen, wie organische Säuren, die unter den pH-Wert-Bedingungen des Blutplasmas in einem Gleichgewicht zwischen protoniertem und deprotoniertem Zustand vorliegen, stärker protoniert und damit das Gleichgewicht zugunsten eines protonierten und fettlöslicheren Zustandes verschoben. Hierdurch wird erreicht, daß mehr solcher saurer Substanzen aus dem Plasma in den Extrakt überführt werden können.

Die kombinierte Messung von ultrazentrifugierten und extrahierten Plasmaproben, sei es nacheinander oder im Gemisch, ist erfindungsgemäß besonders bevorzugt. Die Bestimmung anhand zweier unterschiedlicher Aufbereitungen ein und derselben Plasmaprobe, die in der Regel zu qualitativ und/oder quantitativ unterschiedlichen Erregungsmustern führt, bietet die Möglichkeit, eine größere Anzahl an Standards sowohl für Blut von gesunden Patienten als auch für Blut von Patienten mit unterschiedlichen Krankheitsbildern und Krankheitsschweren mit den zu untersuchenden Blutproben zu vergleichen, wodurch die Zuverlässigkeit der Ergebnisse und die Reproduzierbarkeit erheblich verbessert werden.

Im Rahmen der Arbeiten zu dieser Erfindung wurde im wesentlichen der Effekt von Plasmaproben von Patienten mit metabolischen Encephalopathien auf die elektrische Aktivität neuronaler Netzwerke in vitro untersucht. Die Ergebnisse haben gezeigt, daß Ultrafiltrate und Extrakte derartiger Proben gegenüber Proben von gesunden Patienten eine deutliche Veränderung der Netzwerkaktivität bewirken. Diese korrelierte mit dem Grad der existierenden Encephalopathie des Patienten. Untersuchungen anhand einer Vielzahl von neuronal aktiven Substanzen haben die Dosis- und Substanzspezifität der Netzwerkreaktionen und die sehr gute Reproduzierbarkeit der erhaltenen Ergebnisse am selben und an verschiedenen Netzwerken nachgewiesen. Das System der auf Mikroelektroden-Arrays kultivierten Neuronen bzw. neuronalen Netzwerke, kann somit vorteilhaft zur Diagnostik und Differentialdiagnostik metabolischer Encephalopathien sowie zur Kontrolle bestehender und neuartiger Therapiekonzepte angewendet werden.

Die Anwendung des erfindungsgemäßen Verfahrens ermöglicht als Konsequenz eine verbesserte, zielgerechte Behandlung von Patienten, was wiederum eine Reduktion von Morbidität und Mortalität zur Folge hat. Durch frühzeitige Initiierung einer adäquaten Therapie kann darüber hinaus eine Kostenersparnis durch verkürzte Verweildauer der Patienten im intensivmedizinischen Bereich und im Krankenhaus erreicht werden. Die Identifikation der den Encephalopathien zugrundeliegenden Pathomechanismen mit Hilfe des erfindungsgemäßen Verfahrens könnte darüber hinaus Ansätze zur Entwicklung neuartiger Präventions- und Therapiestrategien schaffen.

### Beispiele

### Ultrafiltration

Die Ultrafiltration der Plasmaproben wurde mit Ultrafiltern der Firma Schleicher & Schüll, Deutschland, für Probenvolumina von jeweils 0,5 ml mit einer Ausschlußgröße der Membran von 30.000 Dalton nach Angabe des Herstellers durchgeführt.

### Extraktion

500 µl einer zu untersuchenden Plasmaprobe wurden in einem 2 ml-Safe-Lock-Reaktionsgefäß (Firma Eppendorf, Hamburg) mit 150 µl Salzsäure (37%, zur Analyse, Merck, Darmstadt) angesäuert. Nach Zugabe von 1000 µl Dichlormethan (zur Analyse, Merck, Darmstadt) wird die Probe unter Verwendung eines Mischers (Gyrator Uniprep, Firma UniEquip, Martinsried) für 3 Minuten intensiv gemischt und anschließend für 5 min. bei 20800 x g zentrifugiert (Zentrifuge 5417 C, Firma Eppendorf, Hamburg). Die untere, organische Phase (Dichlormethanphase) wurde abgenommen, in ein neues 2 ml-Safe-Lock-Reaktionsgefäß überführt und bei Raumtemperatur vorsichtig verdampft. Nach vollständigem Verdampfen des Lösungsmittels wurde der im Gefäß verbliebene Niederschlag in 500 µl einer 1 %igen Dimethylsulfoxid-(DMSO)Lösung in DMEM (Dulbecco's Minimal Essential Medium) wieder in Lösung gebracht. Die Mischung der Probe erfolgte durch erneutes Schütteln in der oben beschriebenen Weise. Die so aufbereitete Plasmaprobe wurde zur Messung mit den Neuronen in Berührung gebracht.

### Herstellung neuronaler Netzwerke auf Mikroelektroden-Arrays - Fetale Maus-Hirn-Kultur / Fetale Ratten-Hirn-Kultur

Die Kultivierung von Neuronen auf Mikroelektroden-Arrays (Neurochips) wurde nach bekannten Verfahren durchgeführt (Gross, G.W., Internal Dynamics of randomized mammalian neuronal networks in culture. In: Enabling Technologies for Cultured Neural Networks (Stenger, D.A. and McKenna, T.M., eds.), Academic Press, New York, 1994, Seiten 277 - 317; DE 199 32 720 A1 von Siebler et al.)

Die Vorbereitung der Sensor-Zellkultur-Oberfläche zum Bewachsen mit Nervenzellkulturen beinhaltet die Sterilisation (z.B. mit Alkohol, UV-Licht, Gamma-Strahlen), eine Hydrophilisierung der Oberfläche (z.B. Abflammen, Säure- oder Lauge-Behandlung mit anschließendem Wässern) sowie das Aufbringen von kationischen Ladungsträgern und/oder Anheftungsfaktoren (einzeln, in Kombination oder im Gemisch, z.B. Polylysin, Polyethylenimin, Laminin, Fibronectin).

Die Hirne oder Teile davon werden von Feten (Trächtigkeitstag 15-18) präpariert. In einer Pufferlösung wird der Zellverband mechanisch mit Skalpellklingen zerkleinert. Anschließend werden durch Zusatz von Verdauungsenzymen Bestandteile der extrazellulären Matrix und Zellmembranproteine angedaut. Die Wirkung der Verdauungslösung wird durch Zugabe eiweißhaltiger Nährlösung gestoppt und das Gewebe wird nach dem Absaugen der geblockten Verdauungslösung in Nährmedium verbracht. Durch vorsichtiges Auf-und-Ab-Pipettieren werden die Zellen vereinzelt.

Dann wird die Zellsuspension abhängig von Tierart und Ursprungsgewebe in einer Dichte von 50.000 bis 1 Mio. Zellen/cm² auf der vorbehandelten Sensor-Zellkultur-Oberfläche der Neurochips ausgesät. Die Neurochips werden in Plastikschälchen mit Deckel gestellt und in einen Brutschrank verbracht (37 °C, Luft mit 5-10 % Kohlendioxid-Zusatz, 90-95 % Luftfeuchte). Zwei- bis dreimal wöchentlich wird ein Teil des verbrauchten Nährmediums durch frisches ersetzt.

Die Zellen (ein Gemisch aus Nervenzellen und Gliazellen, die Stütz- und Stoffwechselfunktionen haben) heften sich an der Sensor-Zellkultur-Oberfläche an, und aus den Nervenzellen wachsen Fortsätze aus und bilden untereinander Verknüpfungen. Dabei besteht ein.enger Kontakt mit den Gliazellen, so daß ein in der Zusammensetzung dem Ursprungsgewebe ähnelndes Nervennetzwerk entsteht. Dieses entwickelt im Verlauf von einigen Tagen bis Wochen ein spontanes elektrisches Aktivitätsmuster, das gewebetypisch ist und durch die Kopplung der Zellen an die Sensoren gemessen werden kann.

### Ausführungsbeispiel

Dissoziiertes Vorderhirnrindengewebe aus 18 Tage alten Mäuseembryos wurde auf Mikroelektroden-Array-Platten mit 64 Dünnfilmelektroden auf einer Fläche von 1 mm² kultiviert. Die Zellen wurden nach dem obengenannten Verfahren von Gross in Kultur gehalten. Von zwei gesunden Probanten (männlich) und vier Patienten im Leberversagen, die einer Leberdialyse mit dem MARS-System unterzogen wurden, wurden Plasmaproben genommen (s. Tabelle 1). Höhermolekulare Proteine wurden mittels Ultrafiltration nach dem oben beschriebenen Verfahren mit einer Ausschlußgröße von 30.000 Dalton entfernt.

**Tabelle 1: Patientencharakteristika und Grad der hepatischen Encephalopathie (HE)**

| Nummer | Geschlecht/Alter | Krankheitsbild | Grad der HE |
|---|---|---|---|
| 1 | weiblich/24 Jahre | Dekompensierte alkoholische Leberzirrhose | Grad III |
| 2 | weiblich/43 Jahre | alkoholische Leberzirrhose, hepatorenales Syndrom | Grad II-III |
| 3 | weiblich/46 Jahre | alkoholische Leberzirrhose, hepatorenales Syndrom | Grad II-III |
| 4 | männlich/44 Jahre | Dekompensierte alkoholische Leberzirrhose | Grad III |

Die Experimente wurden in einer mit einer entfernbaren, beheizten Plastikabdeckung abgedeckten offenen Edelstahlkammer, in der eine Temperatur von 37°C herrschte, in einer Atmosphäre aus Luft mit 10% CO₂ durchgeführt. Die Zellen wurden regelmäßig lichtmikroskopisch überprüft. Für eine verbesserte Dokumentation der morphologischen Veränderungen der Zellen während des Experiments wurde parallel eine zeitabhängige Aufzeichnung durchgeführt. Die elektrische Aktivität der Elektroden wurde verstärkt und digital erfaßt (Software von Spectrum Scientific, Dallas, Texas, USA). Nach dem Aufbau des Experiments wurden die Kulturen für 30 Minuten bis 1 Stunde stehen gelassen, damit sich ihre Hintergrundaktivität einstellen konnte. Danach wurde die spontane Aktivität der neuronalen Netzwerke für wenigstens 30 Minuten aufgezeichnet. Gleiche Anteile von jeweils 100 Mikroliter der Ultrafiltrate des menschlichen Blutplasmas wurden anschließend zu 600 Mikroliter Kulturmedium auf dem neuronalen Netzwerk gegeben und für bis zu 2 Stunden stehen gelassen. Die Kultur wurde anschließend wenigstens zweimal mit Zellkulturmedium gewaschen und die Aktivität der neuronalen Netzwerke erneut für wenigstens 30 Minuten aufgezeichnet, bevor die nächste zu untersuchende Probe zugegeben wurde.

### Beschreibung der Figuren:

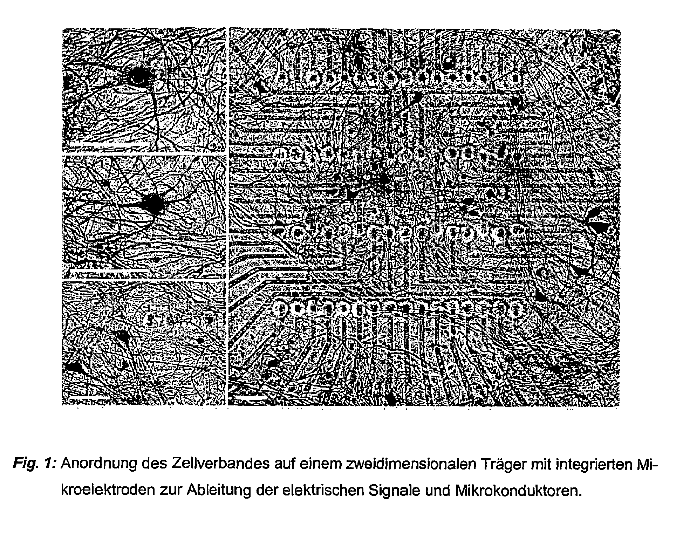

- Figur 1: zeigt eine schematische Darstellung der Anordnung des Zellverbandes auf einem zweidimensionalen Träger mit integrierten Mikroelektroden zur Ableitung der elektrischen Signale und Mikrokonduktoren.

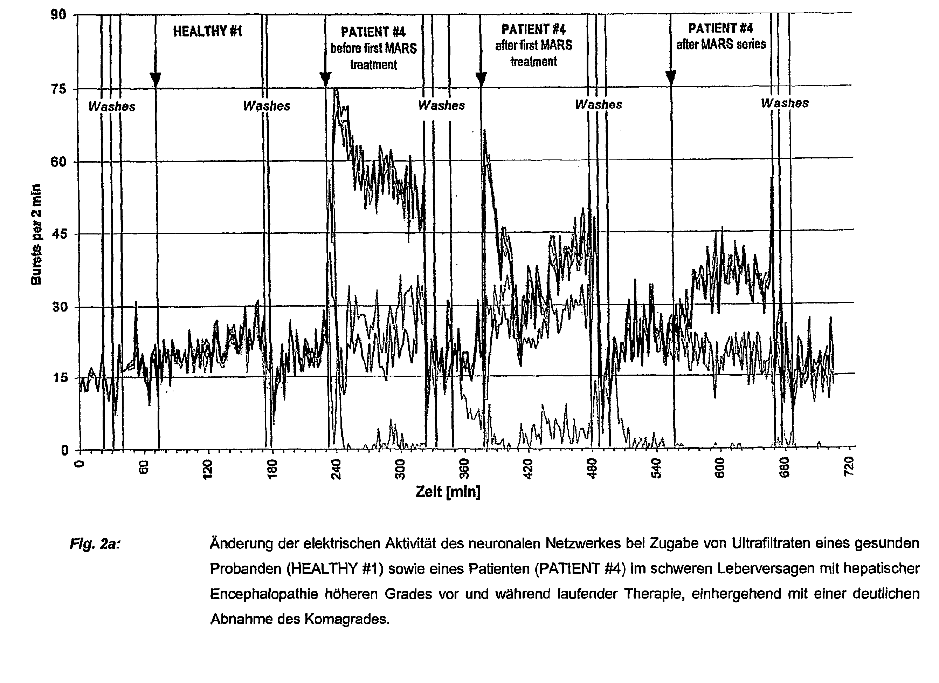

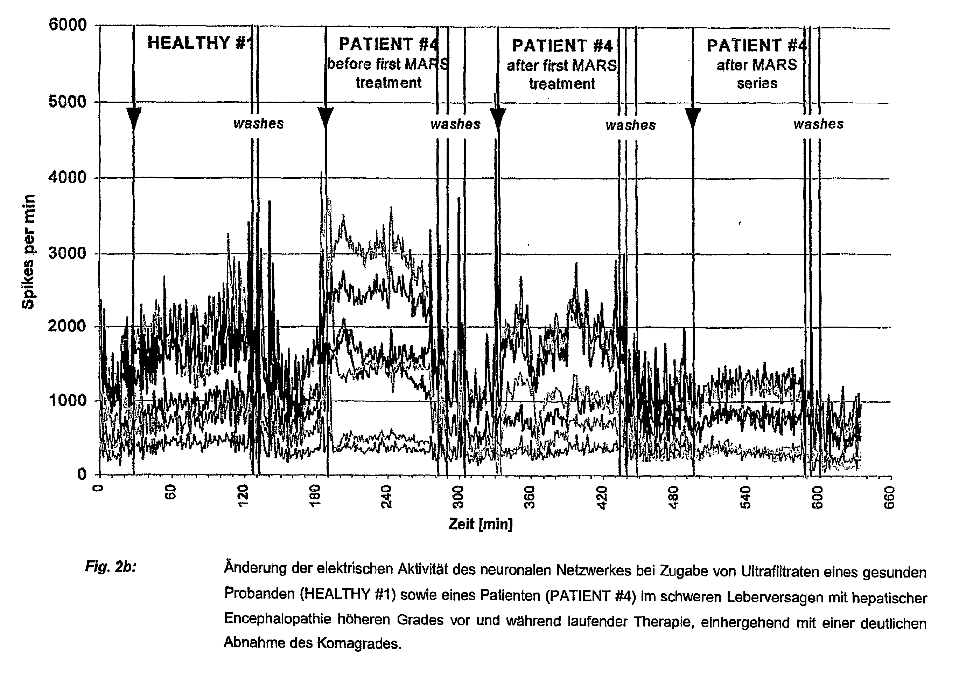

- Figuren 2a und 2b: zeigen die Änderung der elektrischen Aktivität des neuronalen Netzwerkes bei Zugabe von Ultrafiltraten eines gesunden Probanden (HEALTHY #1) sowie eines Patienten (PATIENT #4) im schweren Leberversagen mit hepatischer Encephalopathie höheren Grades vor und während laufender Therapie, einhergehend mit einer deutlichen Abnahme des Komagrades.

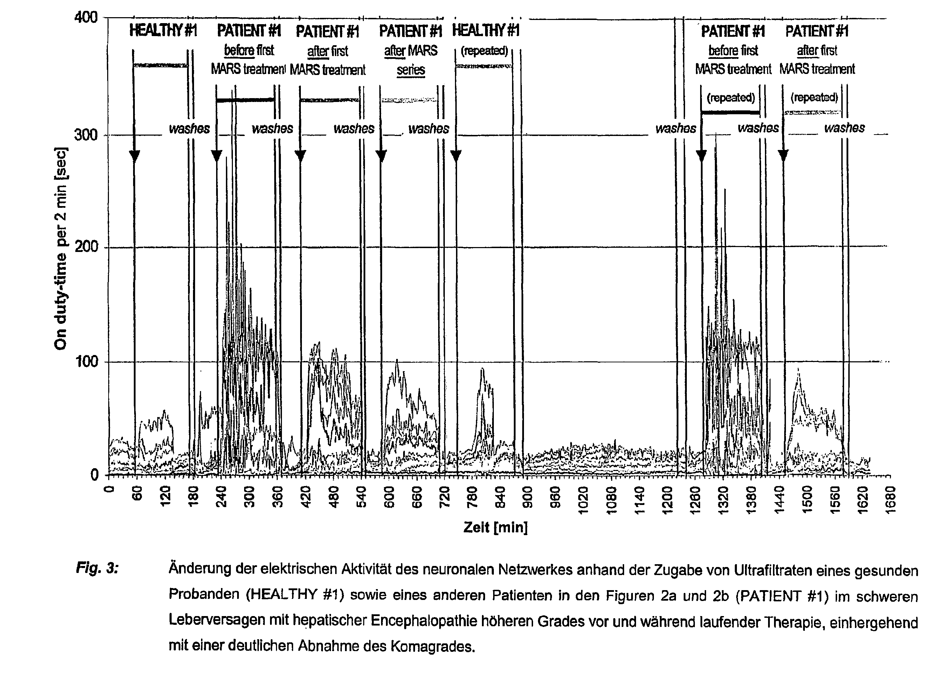

- Figur 3: zeigt die Reproduzierbarkeit der Änderung der elektrischen Aktivität des neuronalen Netzwerkes, wie sie in den Figuren 2a und 2b gezeigt ist, anhand der Zugabe von Ultrafiltraten eines gesunden Probanden (HEALTHY #1) sowie eines anderen Patienten (PATIENT #1) im schweren Leberversagen mit hepatischer Encephalopathie höheren Grades vor und während laufender Therapie, einhergehend mit einer deutlichen Abnahme des Komagrades.

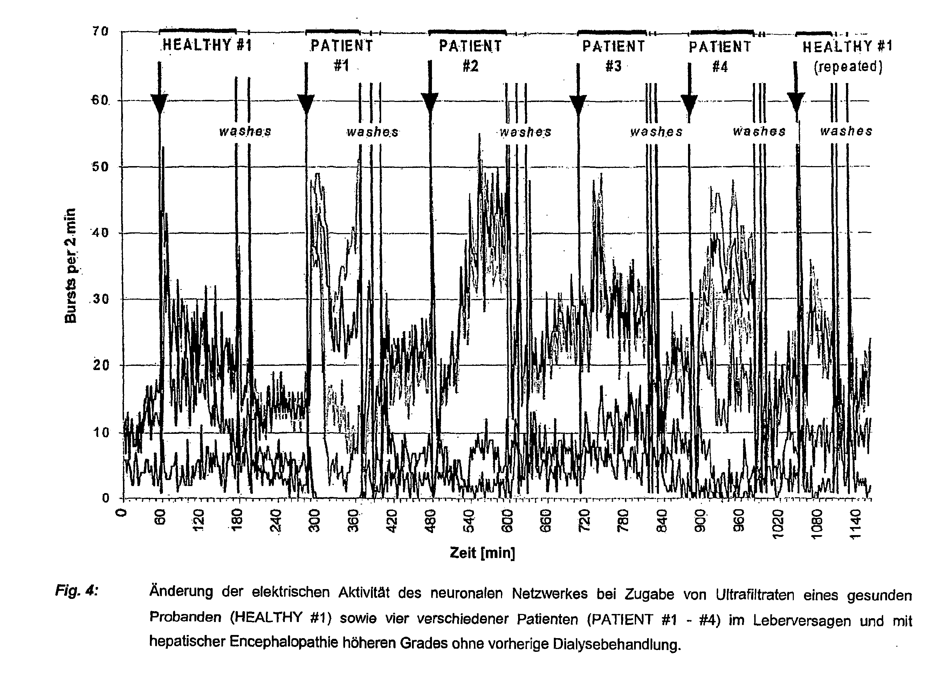

- Figur 4: zeigt einen Vergleich der auftretenden Änderung der elektrischen Aktivität des neuronalen Netzwerkes bei Zugabe von Ultrafiltraten eines gesunden Probanden (HEALTHY #1) sowie vier verschiedener Patienten (PATIENT #1 - #4) im Leberversagen und mit hepatischer Encephalopathie höheren Grades ohne vorherige Dialysebehandlung.

In Figur 1 ist die Anordnung eines neuronalen Zellverbandes zwischen Gliazellen und anderen natürlicherweise im Zentralnervensystem vorhandenen zellulären Elementen (Fibroblasten, Endothelzellen) auf einem Multi-Elektroden-Array dargestellt, wobei die neuronalen Zellen untereinander durch synaptische Verbindungen vernetzt sind und deren Anordnung in unmittelbarer Nähe der in der Trägerplatte vorhandenen Mikroelektroden eine Ableitung der elektrischen Signale der einzelnen Neuronen gestattet.

Die Figuren 2a und 2b zeigen den Effekt von Ultrafiltraten eines gesunden Probanden (HEALTHY #1) sowie eines Patienten (PATIENT #4) im schweren Leberversagen vor, während und nach einer spezifischen Therapie nach dem MARS-Verfahren (Molecular Adsorbents Recirculating-System) zur Entfernung von im Leberversagen akkumulierenden Giftstoffen aus dem Organismus. Bei diesen handelt es sich neben wasserlöslichen Metaboliten auch um relativ lipophile Substanzen. Letztere werden im Blut primär an Plasmaeiweiße - vor allem an Albumin - gebunden transportiert. MARS ermöglicht durch das Vorhandensein von Akzeptorproteinen (Albumin) im Dialysat die Elimination dieser ansonsten kaum dialysablen Verbindungen aus dem Blutplasma. Zudem wird hierdurch auch eine günstige Beeinflussung der ansonsten bei diesem Krankheitsbild typischen Aminosäuredysbalance zwischen verzweigtkettigen und aromatischen Aminosäuren im Plasma erreicht.

Figur 2a zeigt die Abhängigkeit der Burstrate (Bursts pro Zeiteinheit) und Figur 2b die Abhängigkeit der Spikerate (Spikes pro Zeiteinheit) von der Zugabe unterschiedlicher Ultrafiltrate. Ein Spike stellt dabei die registrierte Aktivität von einem neuronalen Aktionspotential dar. Als Burst bezeichnet man die sehr rasche Aufeinanderfolge vieler Aktionspotentiale in einer neuronalen Einheit über einen eng begrenzten Zeitraum in Form von "Spikepaketen". Die in den Diagrammen dargestellten unterschiedlichen Aktivitätsverläufe wurden jeweils von sieben verschiedenen extrazellulären Elektroden eines Multi-Elektrodenarrays während desselben Experimentes registriert. Während sich bei Zugabe der Probe vom Gesunden kaum eine Änderung der Aktivität des Zellverbandes zeigte, kam es nach Zugabe einer in identischer Weise aufbereiteten Probe eines Patienten im schweren Leberversagen mit hepatischem Koma höheren Grades zu einem deutlichen Anstieg der Aktivität mehrerer neuronaler Einheiten, während andere relativ unbeeinflußt blieben und sich zum Teil sogar eine Hemmung der neuronalen Aktivität zeigte. Die identisch aufbereiteten Proben desselben Patienten nach Ende der ersten MARS-Behandlung und nach Abschluß der Behandlungsserie (5 Einzelbehandlungen, jeweils eine Behandlung pro Tag) verursachten geringere Änderungen der Netzwerkaktivität gegenüber dem Zustand vor Beginn des Experimentes. Dabei zeigte sich eine Korrelation mit der Dauer der erfolgten MARS-Behandlung und mit dem klinisch erfaßten Komagrad des Patienten in der Art, daß die ausgelösten Änderungen mit zunehmender Behandlungsdauer und abnehmendem Encephalopathiegrad der Patienten ebenfalls abnahmen. Zwischenzeitliche Waschungen (partieller Wechsel des Mediums) zeigten die Reversibiltät der beobachteten Effekte.

Figur 3 zeigt einen gleichartigen Versuchsablauf, wie in Figur 2 dargestellt, mit Wiederholung der Versuchsabfolge, allerdings anhand eines anderen, jedoch gleichartigen Zellverbandes und unter Verwendung von Proben eines anderen Patienten (PATIENT #1), der sich ebenfalls im hepatischen Koma befand und ebenfalls in gleicher Weise nach dem MARS-Verfahren behandelt wurde. Dabei zeigte sich ein hohes Maß an Reproduzierbarkeit der nach Zugabe der verschiedenen Proben eintretenden neuronalen Aktivitätsänderungen.

Die Änderung der Aktivität von 13 neuronalen Einheiten, gezeigt durch die unterschiedlichen Graphen, ist als Produkt von Burstrate und mittlerer Dauer der Bursts pro Zeiteinheit (in der Figur "on-duty time" genannt) dargestellt. Die Effekte der verwendeten Proben auf die Netzwerkaktivität entsprechen im wesentlichen den zu Abbildung 2 beschriebenen. Man beobachtet eine geringe Änderung der Aktivität nach Zugabe der Probe von einem gesunden Individuum (HEALTHY #1) gegenüber der Ausgangsaktivität des Netzwerkes, dagegen jedoch eine starke Aktivierung der Mehrzahl der erfaßten neuronalen Einheiten nach Zugabe der Probe des Patienten (PATIENT #1) vor Beginn der MARS-Behandlung. Mit zunehmender Dauer der MARS-Behandlung erfolgt eine immer weitere Abnahme der ausgelösten Änderungen und somit eine Annäherung an den Effekt von Proben vom Gesunden. Auch hier zeigte sich eine Korrelation des Ausmaßes des verursachten Effektes der Proben auf die neuronale Aktivität mit dem klinisch auffälligen Encephalopathiegrad des Patienten. Durch Wiederholung der Probenabfolge konnte ein hohes Maß an Reproduzierbarkeit der nach Zugabe der verschiedenen Proben eintretenden neuronalen Aktivitätsänderungen nachgewiesen werden.

Figur 4 zeigt den Vergleich der Effekte von Ultrafiltraten des Blutplasmas von vier verschiedenen Patienten (PATIENT #1 - #4) im Leberversagen mit höhergradiger hepatischer Encephalopathie sowie eines gesunden Individuums (HEALTHY #1, getestet zu Beginn und am Ende des Experimentes) auf die Burstrate von sechs verschiedenen Einheiten eines neuronalen Netzwerkes. Die Änderung der Netzwerkaktivität nach Zugabe der Patientenproben zeigte individuelle Unterschiede, die auf eine unterschiedliche stoffliche Zusammensetzung der Ultrafiltrate schließen lassen. Dabei war der Unterschied zur Probe des gesunden Individuums mehr oder minder ausgeprägt. Dies könnte eine Grundlage für eine Unterscheidung verschiedener Schweregrade der Erkrankung und Vorhandensein oder Nichtvorhandensein von für die Komaentstehung relevanten Begleiterkrankungen sein. In Bezug auf die Burstrate war die nach Zugabe der Proben von Patienten in unterschiedlichem Maße auftretende Aufsplittung der Aktivitätszustände der einzelnen neuronalen Einheiten, in der Figur dargestellt durch die Abstände der Graphen voneinander, das hervortretendste Merkmal (siehe auch Figur 2 a) im Vergleich mit Proben gesunder Individuen.

## Patentansprüche

1. Verfahren zur extrakorporalen qualitativen und/oder quantitativen Erfassung neurotoxischer Substanzen im Blutplasma eines Individuums, bei dem man ein System mit auf einem Mikroelektroden-Array bzw. einem Neurochip kultivierten oder aufgebrachten Neuronen oder neuronalen Netzwerken und einer Vorrichtung zum Messen der elektrischen Aktivität wenigstens eines oder mehrerer der Neuronen bereitstellt, die Neuronen mit einer aufbereiteten Plasmaprobe in Berührung bringt und die elektrische Aktivität der Neuronen mißt,
**dadurch gekennzeichnet, daß**
das Blutplasma des Individuums zur Aufbereitung vor der Messung einer Extraktion fettlöslicher Stoffe unter Verwendung eines organischen Lösungsmittels für fettlösliche Stoffe unterzieht
und das Blutplasma des Individuums zur Aufbereitung vor der Messung gegebenenfalls einer Ultrafiltration unterzieht, wobei eine Ausschlußgrenze für das Molekulargewicht gewählt wird, die eine weitestgehende Abtrennung der Plasmaeiweiße von den anderen gelösten Substanzen bewirkt,
und man den fettlösliche Stoffe enthaltenden Extrakt und gegebenenfalls das Ultrafiltrat im Gemisch mit dem Extrakt oder beides nacheinander für die Messung einsetzt.

2. Verfahren nach Anspruch 1, **dadurch gekennzeichnet, daß** das Filtrationsverfahren mit einer Ausschlußgrenze für das Molekulargewicht von höchstens 45.000 Dalton, bevorzugt höchstens 30.000 Dalton durchgeführt wird.

3. Verfahren nach einem der vorangegangenen Ansprüche, **dadurch gekennzeichnet, daß** man das Blutplasma vor der Extraktion ansäuert, wobei man vorzugsweise eine anorganische Säure, besonders bevorzugt Salzsäure, verwendet.

4. Verfahren nach einem der vorangegangenen Ansprüche, **dadurch gekennzeichnet, daß** man für die Extraktion fettlöslicher Stoffe Dichlormethan oder Chloroform verwendet.

5. Verfahren nach einem der vorangegangenen Ansprüche, **dadurch gekennzeichnet, daß** man die organische Phase nach der Extraktion in ein separates Gefäß überführt, das organische Lösungsmittel durch Verdunsten entfernt, die zurückbleibenden extrahierten fettlöslichen Substanzen durch Hinzufügen einer geeigneten Flüssigkeit, vorzugsweise einer Mischung aus Zellkulturmedium und einer geringen Konzentration eines Lösungsvermittlers, wie Dimethylsulfoxid, wieder in Lösung bringt.

## Claims

1. A method of extracorporal qualitative and/or quantitative detection of neurotoxic substances in the blood plasma of an individual, in which there is provided a system with neurons or neuronal networks applied or cultivated on a microelectrode array or a neurochip and a device for measuring the electrical activity of at least one or more of the neurons, the neurons are brought into contact with a processed plasma sample and the electrical activity of the neurons is measured,
**characterized in that**
the plasma of the individual is subjected to an operation for the extraction of fat-soluble substances using a solvent for fat-soluble substances, for preparation prior to the measurement operation,
and optionally the blood plasma of the individual is subjected to an ultrafiltration operation, for preparation prior to the measurement operation, wherein an exclusion limit is selected for the molecular weight, which provides for very substantial separation of the plasma proteins from the other dissolved substances,
and the extract containing fat-soluble substances, and optionally the ultrafiltrate in admixture with the extract, or both in succession are used for the measurement operation.

2. A method as set forth in claim 1 **characterized in that** the filtration process is carried out with an exclusion limit for the molecular weight of at most 45,000 Daltons, preferably at most 30,000 Daltons.

3. A method as set forth in one of the preceding claims **characterized in that** the blood plasma is acidified prior to the extraction operation, preferably using an inorganic acid, particularly preferably hydrochloric acid.

4. A method as set forth in one of the preceding claims **characterized in that** dichloromethane or chloroform is used for the extraction of fat-soluble substances.

5. A method as set forth in one of the preceding claims **characterized in that** after the extraction operation the organic phase is transferred into a separate vessel, the organic solvent is removed by evaporation, and the extracted fat-soluble substances which have remained behind are re-dissolved by the addition of a suitable liquid, preferably a mixture of cell culture medium and a low concentration of a dissolving intermediary such as dimethyl sulfoxide.

## Revendications

1. Procédé pour la détection extracorporelle qualitative et/ou quantitative de substances neurotoxiques dans le plasma sanguin d'un individu, dans lequel on prépare un système avec des neurones ou réseaux neuronaux cultivés ou implantés sur un rang de microélectrodes ou une neuropuce et un dispositif pour mesurer l'activité électrique d'au moins un ou plusieurs neurones, met les neurones en contact avec un échantillon de plasma préparé et mesure l'activité électrique des neurones, **caractérisé en ce que** le plasma sanguin de l'individu subit pour la préparation avant la mesure une extraction de matières liposolubles en utilisant un dissolvant organique pour matières liposolubles, et le plasma sanguin de l'individu subit le cas échéant pour la préparation avant la mesure une ultrafiltration, une limite d'exclusion étant définie pour le poids moléculaire, laquelle opère une séparation très poussée des protéines plasmatiques des autres substances dissoutes, et utilise l'extrait contenant les matières liposolubles et le cas échéant l'ultrafiltrat mélangé à l'extrait ou les deux l'un après l'autre pour la mesure.

2. Procédé selon la revendication 1, **caractérisé en ce que** le procédé de filtration est effectué avec une limite d'exclusion pour le poids moléculaire de 45 000 Dalton au maximum, de préférence de 30 000 Dalton au maximum.

3. Procédé selon l'une des revendications précédentes, **caractérisé en ce que** l'on acidifie le plasma sanguin avant l'extraction, utilisant de préférence un acide anorganique, en particulier de préférence de l'acide chlorhydrique.

4. Procédé selon l'une des revendications précédentes, **caractérisé en ce que** l'on utilise du dichlorométhane ou du chloroforme pour l'extraction des matières liposolubles.

5. Procédé selon l'une des revendications précédentes, **caractérisé en ce que** l'on transfert la phase organique après l'extraction dans un récipient séparé, élimine le dissolvant organique par évaporation et remet en solution les substances liposolubles extraites restantes par adjonction d'un liquide approprié, de préférence un mélange composé d'un milieu de culture cellulaire et d'une faible concentration d'un agent de solubilisation, tel que sulfoxyde diméthylique.
